# EUROPEAN PATENT APPLICATION

(11) **EP 2 548 607 A2**
(43) Date of publication of application: **23.01.2013**
(21) Application number: 12176943.4
(22) Date of filing: 18.07.2012
(51) Int. Cl.: A61M 25/10, A61B 5/00

(54) **Micro foam sclerosing catheter for creating a spatially separated vessel section and for generating micro foam inside the catheter**

(30) Priority: 20.07.2011 EP 11174690
(71) Applicant: Netzer, Florian, 80803 München (DE)
(72) Inventor: Netzer, Florian, 80803 München (DE)
(74) Representative: Maiwald Patentanwalts GmbH

(57) **Abstract**

The present invention relates to a catheter and a corresponding method to use a catheter, wherein the catheter provides for two inflatable balloon devices which are configured to perform a relative movement with respect to each other. By uncovering an opening upon distancing the two inflated balloon devices, a confined and spatially separated vessel section is created in which optical energy may be emitted to treat the vessel. The presented catheter advantageously enables the user to generate micro foam for sclerosing application inside the catheter.

## Description

### FIELD OF THE INVENTION

The present invention relates to catheter technology. In particular, the present invention relates to a catheter for creating a spatially separated vessel section and a method for creating a spatially separated vessel section.

### BACKGROUND OF THE INVENTION

In medicine, a catheter is a tube that can be inserted into a body cavity, duct or vessel. Catheters thereby allow drainage, administration of fluids or gases, or access by surgical instruments. In most uses, a catheter is a thin, flexible tube and may be called a soft catheter. In some other uses, it is a larger, solid catheter which may be named a hard catheter. Moreover, a balloon catheter is known in the art as a type of soft catheter with a single inflatable balloon at its tip which is used during a catheterization procedure to enlarge a narrow opening or passage within a body. The deflated balloon catheter is positioned, then inflated to perform the necessary procedure, and deflated again in order to be removed. By means of mechanical pressure, narrow passages within a vessel may be spatially broadened due to the applied force of the inflated balloon.

In case a balloon catheter is inserted into a blood vessel and the user desires to insert specific surgical instruments via the catheter, the application of said surgical instruments will always take place within a vessel which is full of blood. However, certain surgical instruments or certain applications may suffer from disadvantages caused by the presence of the blood at the desired position.

In prior art devices the foam is created far remote from its point of application and before it enters the human body. The foam - especially foam that has been created by using an liquid therapeutic drug of low concentration - is partially or even totally collapsing on its way to the point of application, especially when a long distance has to be bridged, there is the problem occurring that it is difficult to impossible to administer such low concentrated foam efficiently so far. However, in micro foam sclerosis applications the usage of foam with low dose and low concentration of the therapeutic agent for sclerosis is desirable, as it causes less side effects compared to a higher dose micro foam.

### SUMMARY OF THE INVENTION

It may be seen as an object of the present invention to provide for an improved catheter technology.

The object of the present invention is solved by the subject-matter of the independent claims. Further advantages and embodiments are incorporated in the dependent claims.

It should be noted that the invention in the following will be described with regard to a catheter and thus will be described with respect to apparatus type claims. However, features and advantages described in context of the catheter do similarly apply and relate to the below presented method for generating a micro foam inside of a catheter, and vice versa.

It should also be noted that in the context of the present invention the term catheter is synonymously used with the term micro foam sclerosing catheter for creating a spatially separated vessel section and for generating a micro foam inside the catheter when describing the catheter of the present invention.

According to an exemplary embodiment of the invention, a micro foam sclerosing catheter for creating a spatially separated vessel section and for generating a micro foam inside the catheter is presented. The catheter comprises a tube for inserting into a blood vessel, a first balloon device and a second balloon device. Furthermore, at least one of the first balloon device and the second balloon device is movably arranged at the tube. Moreover, the movable arrangement of the at least one of the first balloon device and the second balloon device enables for a change of distance between the first balloon device and the second balloon device. Consequently, the desired separated vessel section can be established i.e. the spatial separation can be completed within the vessel. Moreover, the catheter comprises a micro foam device for generating a micro foam inside the catheter.

Thus, a double balloon micro foam sclerosing catheter for creating a spatially separated vessel section is presented, wherein the catheter is configured to generate the desired micro foam by itself. This may replace a foam generation at a position distanciated from the application. The presented catheter allows for applying and/ or releasing a micro foam for sclerosing applications.

The catheter of the present invention avoids that especially foam that has been created by using a liquid therapeutic drug of low concentration is partially or even totally collapsing on its way to the application. Therefore, the present invention allows combining a longer catheter with micro foam sclerosis application. The presented catheter and the catheter tube facilitate the administration of low concentrated foam efficiently far from where the catheter enters the body. By generating the foam on the way of the gas and liquid mixture along the catheter, if desired with a maximum shortly before the tip of it, the invention enables the therapist to use less toxic low concentrated foam even on spots in a vessel that are far away from the spot of puncture. Thus, the presented catheter and catheter tube avoid the need to create and administer higher concentrated foam and enables the therapist to use smaller volumes of foam since it reaches the spot of treatment in the optimum quality of the bubbles and therefore reduces the risk of undesired side effects such as nausea, transient blindness, headache, migraine, stroke, etc.

Furthermore, the synergetic effect of the feature and function of the double balloon aspect and the aspect of the micro foam device inside the catheter will be explained in and elucidated with the following explanations.

In the context of the present invention the micro foam device may be seen as a device which facilitates the production of the micro foam close to the position of the application of the foam, namely in the catheter. This is of general advantage for micro foam application, as a huge decrease of vesicles or blebs in the micro foam during a period of foam transport may be avoided. Further, this may be of special advantage in case a low dose of the therapeutic agent for sclerosis is used.

It should be noted that in the context of the present invention, the term liquid is used for therapeutic agent for sclerosis. Thus, the presented catheter provides for micro foam device which mixes a therapeutic agent for sclerosis with a gas, like e.g. surrounding air, to generate the desired micro foam in the catheter.

The inventor of the present invention, inter alia, found the following. Low dose sclerosis micro foam under certain conditions may decay or decompose faster compared to higher dose micro foam. However, it is desirable to make use of low dose sclerosis micro foam due to its decreased side effects on the body of the subject. The herein presented catheter makes use of this insight and provides for a compact solution for generating micro foam close to the application position of the foam. For example, 0,25%-, 05%-, 1%-, 2%- and/or 4%-dosed foam may be generated by the catheter of the present invention. Also other dosed foams may be generated by the presented catheter and/or catheter tube.

The presented catheter may be applied in combination with a therapeutic agent for sclerosis like e.g. Polidocanol, Athoxysklerol®, or the like. Also other compounds may be used, which are known to the skilled person in the art.

As the presented catheter itself generates the micro foam, long transport paths for the micro foam can be avoided. Thus, a decay or decomposition of the micro foam can advantageously be avoided. The micro foam device may be embodied in various ways. For example, the micro foam device may be embodied as protrusions for generating turbulences in the therapeutic agent-gas mixture, as an electromechanical device which sucks in the therapeutic agent-gas mixture, i.e. the liquid gas mixture, and releases the micro foam into a tube of the catheter, as a filter or a sieve, which respectively causes turbulences in the therapeutic agent-gas mixture flowing through the catheter, e.g. a catheter tube. However, also other forms of micro foam device for generating a micro foam inside the catheter are possible.

Various exemplary embodiments herein provide for a specific teaching about different aspects of such a micro foam sclerosing catheter for creating a spatially separated vessel section and for generating a micro foam inside the catheter. In particular, details will be explained regarding the micro foam device, its position, structure, function and advantages. The aspects described above can be combined in different variations with the following aspects of the catheter.

Further, in the context of the present invention the distance between the two balloon devices may be caused by a distancing mechanism. As will be described below several possibilities may be used to embody said distancing mechanism. For example a tubular system comprising three coaxially tubes may be used as defined below.

The tube of the catheter may extend between the two balloon devices. Furthermore the catheter may be embodies as a tubular system comprising three tubes, wherein the first tube is inside of the second and the third tube, whereas the second tube is inside the third tube. Thus, the first tube may be seen as the inner tube, the second tube may be seen as the medium tube and the third tube may be seen as the outer tube.

In such a tubular catheter system the first tube is embodied as a laser catheter. The second tube may comprise the first balloon device and the third tube may comprise the third balloon device. The three tubes may be embodied coaxial such that a relative translation of the tubes with respect to each other is possible by means of e.g. shifting at least one of the tubes. Consequently, the three tubes have three different diameters. If desired, the respective balloon devices may be positioned at the end of the respective tube. By means of translating the third tube relative to the second tube the two balloon devices may be brought in the desired distanced position as described above and below hereinafter. In case of such a tubular system, the opening of the catheter used to release a medium into the treated blood vessel may be comprised by the second tube. The second tube may be shifted or displaced within the third, outer tube such that the opening is uncovered upon shifting the second tube relative to the third, outer tube. If desired, such a tubular catheter system may be configured to simultaneously shift the first and the second tube relative to and within the third tube.

Furthermore, in this and in every other embodiment of the invention the tube may be of transparent material with regard to the wavelength of light which is used. In other words the tube and the wavelength of light which is used may be adapted accordingly according to the present invention.

The spatially separated vessel section may be seen as a vessel section that is spatially confined by the distanced first and second balloon devices and the surrounding walls of the vessel.

In other words, the first and the second balloon device may be attached to the tube of the catheter in such a way that they may be e.g. pulled away from each other into a distanced position. The first balloon device and the second balloon device may e.g. also distanced from another by using the catheter when they are already in an inflated state. If desired, the presented catheter may be inserted into, for example, a blood vessel when the first and second balloon devices are not inflated. After having directed the catheter to its desired location, the first and second balloon devices may be inflated by means of e.g. using an additionally provided medium supply line. Therein, the term "medium" shall be understood in the context of the present invention as gas or liquid or steam of an element or of a composition. This detail will be described hereinafter in more detail.

After the first and second balloon devices are inflated, a distancing mechanism provided by the catheter may be used in order to change especially enlarge the distance between the inflated first and second balloon devices. Such a distancing mechanism may be provided e.g. by translating one of the tubes in a tubular system, as already described above. By using such a distancing mechanism, a spatially separated vessel section is created within the blood vessel by the presented catheter. Such a spatially separated vessel section may then be present as spatially confined by the walls of the blood vessel and the inflated first and second balloon devices. Therefore, a spatial isolation of the vessel section from the remaining vessel parts can be realized by means of distancing the two balloon devices. Said two balloon devices may be filled with a medium like a gas or a fluid through the catheter.

By providing the ability of the presented catheter to allow for a relative movement of the first and second balloon devices with respect to each other, a blood-free zone, the spatially separated vessel section, can be generated by the presented catheter. In sequential steps, the first and second balloon devices may firstly be inflated and afterwards may secondly be distanced from each other when being positioned in the desired vessel.

In the context of the present invention, the term "blood vessel" exemplarily describes a desired destination of the catheter. If the user desires to position the catheter in an organ, artery, vein, or any other cavity comprised by a human body, he may also use the catheter and the comprised tube according to the present invention.

Especially in the case of two or more tissue layers being positioned adjacently close together, the present invention provides for a simple possibility to create an inflated tissue free section of the body. For example a tissue free vessel section may be created between the lungs and the diaphragm by means of the present invention.

Especially in case of malfunctions of venous valves, the presented catheter may provide for special advantages. For example, in case of a treatment of the region around venous valves with optical energy provided by e.g. an optical fiber which guides laser light to such venous valves and/or the walls of the vein, a spatially separated vein section is highly desirable. This desire is met by the presented catheter as the first balloon device and the second balloon device are attached to the tube in such way that the attachment allows for a change of distance between the first balloon device and the second balloon device in an inflated stated when they are positioned in the vein.

According to another exemplary embodiment of the invention the catheter comprises a flushing channel for flushing and/or sucking off media from the separated vessel section.

The flushing channel may provide for an extra and separated volume within the catheter for securely transporting media from the separated vessel section at the application end of the catheter towards the opposing end of the catheter where the clinician is grasping the catheter. A secure media transport in the other direction is also fascilitated.

Due to the presence of the flushing channel for flushing and/or sucking off media therapeutic agents may be used which would cause damage to the patient if it would diffuse into the body after the application. This can be avoided by means of the flushing channel as the separated vessel section may be cleaned during which residual material like residual micro foam, or any other liquid or gas used for therapy, can securely be removed. This is an advantage of the combination of the double balloon feature and the micro foam generating feature. The presented catheter advantageously reduces side effects of a sclerosis application as only in the separated vessel section the therapeutic agent is applied. After the treatment, the remaining media like residual micro foam may be sucked away such that it does not diffuse further into the subject. Thus, higher concentrations of the therapeutic agent may be used for sclerosis without additional side effects. This may also reduce the treatment time and save time for the patient and the clinician.

According to another exemplary embodiment the catheter is operatively connected to a flushing device and or to a sucking device such that a flushing and or sucking of material from the separated vessel section through the flushing channel are facilitated.

According to another exemplary embodiment of the invention the micro foam sclerosing catheter is embodied as a tubular system comprising three tubes, wherein the first tube is inside of the second and the third tube, whereas the second tube is inside the third tube, and wherein the a micro foam device for generating the micro foam in the catheter is positioned in the first tube.

According to another exemplary embodiment of the invention the micro foam device comprises a first opening into which a liquid and/or gas is guidable and a second opening configured to release the generated micro foam towards the created spatially separated vessel section from the catheter. Further, the micro foam device comprises a flow channel between the first and second opening; and the flow channel is configured to generate the micro foam between the first and second opening.

Thus, in a further embodiment a system is presented in which the catheter is connected to a gas source and a liquid source for guiding both media into the catheter to produce the micro foam therein. Therein, the liquid is to be seen as a therapeutic agent for sclerosis. Supply lines to both sources may be used, which may be embodied in various ways. If desired, the catheter may provide for an integral source of therapeutic agent for sclerosis and/or an integral source of gas. Thus, a chamber with a therapeutic agent for sclerosis may be provided and/or a chamber with gas may be provided by the catheter itself. In any case, the catheter is configured such that both media are guidable to the flow channel where they are mixed by the micro foam device.

According to another exemplary embodiment of the invention the micro foam sclerosing catheter comprises a pump device which is operatively connected to the catheter such that a pressure is applicable by the pump device to the flow channel for transporting a liquid gas mixture through the catheter and for generating the micro foam.

If desired, the pump device may also be integral part of the catheter.

According to another exemplary embodiment of the invention the flow channel comprises a plurality of protrusions configured to create turbulences in a liquid gas mixture flowing through the flow channel.

Thus, the protrusions cause turbulences in the therapeutic agent-gas mixture such that the micro foam is generated. The protrusions may be integrally formed with the catheter or may also be physically separated and affixed to the catheter.

According to another exemplary embodiment of the invention the protrusions are configured to cause turbulences to the liquid gas mixture such that the micro foam is generated out of the flowing liquid gas mixture.

According to another exemplary embodiment of the invention a distance of adjacently positioned protrusions in the flow channel decreases in a direction from the first to the second opening

In other words, the distance of adjacently positioned protrusions in the flow channel decreases in the direction of the flow direction in the flow channel. Only two pairs of adjacent protrusions may fulfill this criterion but also a large plurality of pairs of protrusions may fulfill this criterion. Therein, it is possible that the foam generation is continuously increased along the flow channel.

This embodiment may continuously increase the intensity of the generated turbulences leading to a corresponding increase of the foam generation towards the tip of the catheter. Moreover, the flow resistance may be kept relatively small compared to a homogenous distribution of protrusions. The user may individually adapt the protrusion distribution.

According to another exemplary embodiment of the invention the protrusions are embodied as protrusions which radially protrude from an inner tube surface of a tube of the catheter.

This inner tube may be seen as the first tube described herein with respect to a tubular system. But also other configurations are possible in which the protrusions protrude radially inwards from an inner tube surface of a tube of the catheter.

According to another exemplary embodiment of the invention the catheter comprises a first end configured to be grasped by a clinician and a second end for releasing the micro foam into the created spatially separated vessel section. Moreover, the micro foam device is positioned at the catheter at a distance from the second end which distance is between 0 meters to 0,4 meters.

This geometry has been shown to be specifically practical for the clinician. The stable and reliable micro foam is generated and applicable between the two balloons of the catheter.

According to an exemplary embodiment of the invention, the tube comprises a longitudinal axis and at least one of the first balloon device and the second balloon device is movable along the longitudinal axis of the tube.

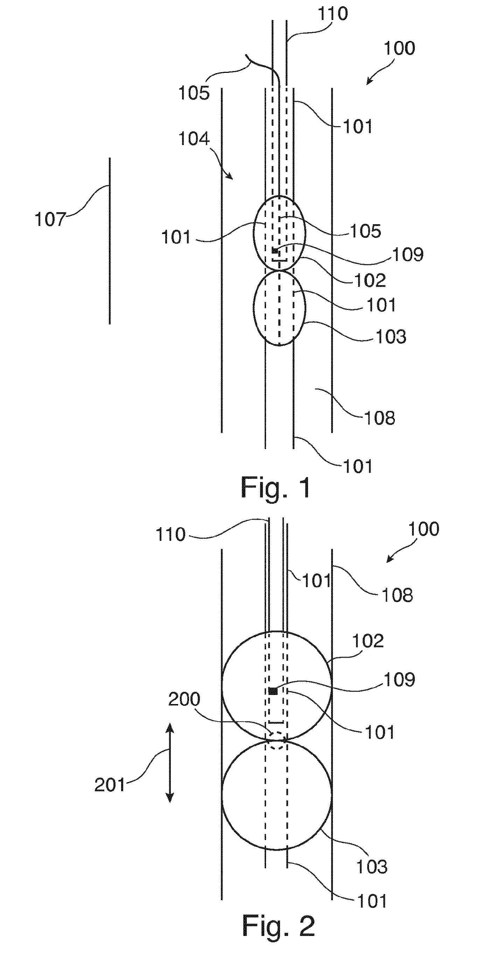

Such a longitudinal axis may be gathered from the following Fig. 1 and the herein presented description of Fig. 1. In other words, the presented embodiment allows for a translational movement of at least one of the first and second balloon device, if the catheter is placed within a blood vessel. If desired, one or also both balloon devices may be inflated before being translated along the longitudinal axis of the tube, such that the spatial confinements, i.e. the walls of the balloon device push out, displace, or suppress blood within the blood vessel when the balloon device is translated along the tube. The previously described procedure allows for establishing a blood-free vessel section or an inflated tissue free section of the body which is spatially separated as two balloon devices may be inflated to such an extent that they spatially adjoin or border directly to the inner surfaces of the blood vessel wall. In other words, when being inflated and distanced from each other, the two balloon devices prevent a blood flow into the vessel section that is located between the two distanced balloon devices.

The presented catheter may allow to separately and independently moving the first and the second balloon device with the moving mechanism i.e. the distancing mechanism. But also the embodiments are comprised by the present invention in which only one of the balloon devices is movable and the respective other one is fixed in its position at the tube.

According to an exemplary embodiment of the present invention, the catheter is also for treating a blood vessel with optical energy, and the tube is adapted to receive an optical fiber. Furthermore, the tube comprises an opening for releasing optical energy of the received optical fiber from inside the tube towards the blood vessel in which the tube is inserted. Especially in case of malfunctions of venous valves, such an optical catheter enables the user to treat the vessel in the spatially separated vessel section by means of optical energy, which is especially advantageous. If desired, the catheter may provide for a radial emission of the electromagnetic energy in order to treat the spatially separated vessel section in a uniform and isotropic way. Therefore, the optical fiber may be comprised by the catheter. Furthermore, the catheter may allow for a rotation of the optical fiber within the catheter such that by rotating the optical fiber, a circular emission of the laser light coupled to the fiber towards the vessel wall is provided. The person skilled in the art directly and unambiguously derives from the above described details that several different optical fibers and several different light sources may be combined in combination with the presented catheter.

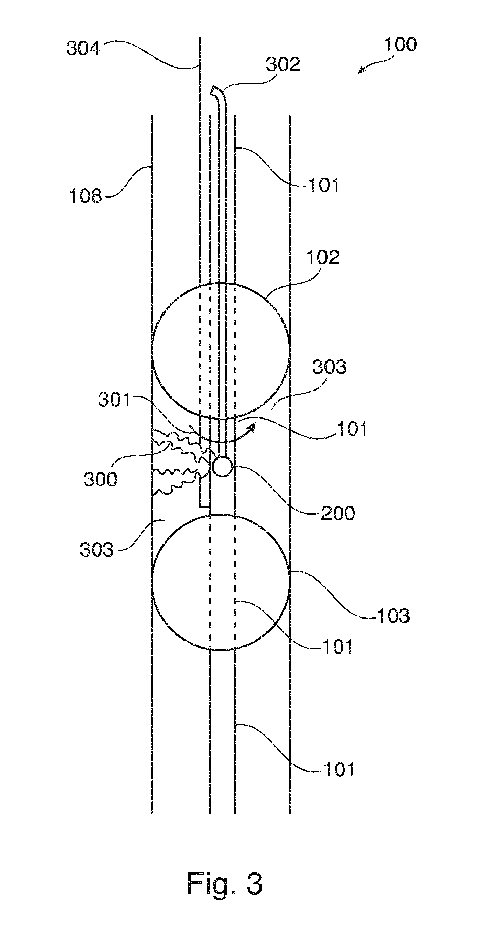

According to an exemplary embodiment, the first and second balloon devices are movably arranged at the tube in such a way that upon an increase of said distance between the first and the second balloon devices, the first and second balloon devices are positioned at opposing positions of the opening for releasing optical energy. As can be, for example, gathered from Fig. 3, the catheter allows for a translation of the two balloon devices along the longitudinal axis upon which translation the opening 200 is uncovered. In other words, the catheter allows for an uncovering of the opening for releasing optical energy by means of causing or forcing at least one of the first and second balloon devices away from its adjacent position from the respective other balloon device. If desired, also both balloon devices may be forced to translate in opposite directions on order to uncover the opening and to simultaneously create the above and below described spatially separated vessel section.

According to an exemplary embodiment, at least one medium supply line like a gas and / or fluid supply line is provided by the catheter. The gas supply line may be directed to the first balloon device and the second balloon device. For example, the catheter may comprise one medium supply line for both balloon devices. In another embodiment two separate supply lines for the two balloon devices may be comprises. In any case the catheter may be configured in combination with the supply line or the two supply lines such that a separate inflation and/or deflation of the balloon devices is possible. If desired a corresponding gas, steam and/or fluid storage device may be comprised in this and every other embodiment of the present invention. In order to inflate the first and second balloon device after they were inserted into the desired blood vessel, a directing of gas along the catheter to the balloon devices is possible and provided by the present invention.

According to another exemplary embodiment of the invention, the first balloon device and the second balloon device are inflatable by supplying gas to the first and the second balloon device via the gas supply line.

According to another exemplary embodiment of the invention, the catheter comprises an optical fiber for guiding optical energy of a light source through the catheter and to release the optical energy in the separated vessel section, a detector for detecting the optical energy, which is released in the separated vessel section. the catheter further comprises a control unit for receiving a detector signal generated by the detector, wherein the control unit is configured to control a light source based on the received detector signal. If desired, the catheter may also comprise a light source like e.g. a laser. The laser may be single wavelength laser or may also be embodied as broadband source. The light source may also be embodied as a pulsed laser. The light source may also be embodied as a tunable light source, being tunable with respect to wavelength, polarization, power, pulse form and / or pulse duration. Said control unit may be connected by wire or wireless with the detector and the light source. In other words a catheter for laser treatment of a blood vessel is presented which comprises a feedback loop or feedback line with regard to the released optical energy. Therein the term "released optical energy" shall be understood as the electromagnetic energy which is emitted in the blood vessel. Therefore, the user is provided with a control of the energy, to which the blood vessel is exposed. This may ensure medical and / or safety requirements.

Especially when releasing a medium like a gas or a liquid into the blood vessel via the catheter, the detector provides for a control signal. The type and the amount of the medium, which is released inside the blood vessel, may change the amount of optical energy that is absorbed by the blood vessel as the optical properties of the propagation path of the light are controllably changed. Hence based on the signal generated by the detector the inflow and outflow of the medium may be controlled e.g. by means of the control unit. The detector may be configured to detect the amount of optical energy which is emitted into the blood vessel.

If desired, a predetermined and desired value of the optical energy that is to be released may be defined such that a regulation of the optical power of the light source is provided to converge to the desired value based on the signal generated by the detector of the catheter. The control unit may be configured to perform such a regulation. For example a desired value may be 75 kJ/cm² per wall of vene. However, the scope of the present invention is not left, if other values are chosen as this value is only exemplary. However, if desired the detector of the catheter may also be configured to detect other physical parameters like wavelength, polarization, power, pulse form and / or pulse duration may be controlled by means of the present invention by inserting a medium like gas or a liquid. Therefore, a control device may be comprised by the catheter of the present invention which is configured to control said physical parameters like e.g. the absorption rate at the vessel or tissue by means of for example a detector. In other words the present invention allows for the generation of a separated vessel section in which the optical properties of the propagation path of the light are controllably changed in order to provide for a improved vessel or tissue treatment.

According to another exemplary embodiment of the present invention the catheter comprises a steam supply line. The steam supply line may be connected with the opening of the tube of the catheter such that the catheter is configured to apply steam to the blood vessel. Steam may be used in order to shrink the diameter of the treated blood vessel according to the present invention. Furthermore, if desired, the steam may be applied such that the blood vessel like e.g. a vene is completely closed by means of steam application through the catheter. The steam supply line may be arranged at the catheter like e.g. the fiber 105 shown in fig. 1 and as is described above and hereinafter in detail. If desired, the catheter of the present invention may comprise a steam generating device and / or a steam storage device. However, also other arrangements of such a steam supply line are possible.

According to another exemplary embodiment of the present invention, the first balloon device and the second balloon device are movable from a minimal distant position to a maximal distant position, and the first and the second balloon devices are positioned adjacently to each other at the tube when they are in the minimal distant position. As may be seen in Fig. 1, a minimal distant position provides for direct contact between the first and the second balloon device. In a maximal distant position, which may be seen in Fig. 3, the balloon devices are positioned at opposing positions of the opening. Thereby, the spatially separated vessel section is created and an uncovering of the opening for emitting for example optical energy or for example ultrasound energy is provided. Thereby space for other surgical instruments as e.g. endoscopes is advantageously generated.

According to another exemplary embodiment of the invention, the catheter is adapted to allow for sequentially inflating and distancing of the first and second balloon devices in such a way that firstly the first and the second balloon devices are inflatable in an adjacent position, and is further adapted in such a way that secondly the distance between the first and second balloon device is adjustable in an inflated state. This process may be gathered from the below described Figs. 1 to 3. However, the present invention may also allow to firstly translate the balloon devices to a desired position, afterwards inflate the balloon devices, and if desired an additional translation or position of the balloon devices may formed by means of the provided catheter.

According to another exemplary embodiment, the catheter further provides for a second opening for releasing a medium like e.g. a gas or a liquid into a vessel section that is spatially confined by the distanced first and second balloon devices.

In particular, the catheter is configured such that the generated micro foam may be released via said opening. By means of providing for a first and second opening, optical energy may be released simultaneously to the release of, for example, an inert gas in order to improve the vessel treatment or tissue treatment by optical energy. Inert gas and / or fluid may be used in this and also in any other exemplary embodiments of the invention in order to reduce chemical reactions taking place during application with the catheter according to the present invention. Furthermore physical parameters like optical absorption may be controlled by means of the present invention by inserting a medium like gas or a liquid. Therefore, a control device may be comprised by the catheter of the present invention which is configured to control said physical parameters like e.g. the absorption rate at the vessel or tissue by means of for example a detector. For example, the optical treatment of the vessel in the spatially separated vessel section may be improved as inert gas may be directed through the catheter into such a spatially separated vessel section.

According to another exemplary embodiment of the invention a catheter tube for generating a micro foam inside a catheter is presented. The catheter tube comprises micro foam device for generating a micro foam inside the catheter. Therein, the above and below described aspects regarding the catheter and the micro foam device can equally be applied to the presented catheter tube.

It should be noted that the term catheter tube and tube may be used synonymously within the context of the present invention.

According to another exemplary embodiment of the invention the use of an inflatable double balloon catheter for generating a micro foam inside the catheter is presented.

According to another exemplary embodiment of the invention a method for generating a micro foam inside of a catheter is presented. The method comprises the steps of providing for a micro foam sclerosing catheter comprising a micro foam device for generating a micro foam inside the catheter, guiding a liquid gas mixture trough the micro foam device of the catheter and thereby generating the micro foam.

According to another exemplary embodiment of the invention, the method provides for emitting a medium like a micro foam, a gas or a fluid in the separated vessel section through the tube.

This method step may be performed by means of a second opening provided by the tube. However, the gas may also be inserted into the separated vessel section through the first opening through which also the optical energy is emitted. If desired, inert gas or a fluid may used in order to improve the application of optical energy to the vessel section.

In this and every other embodiment of the invention the insertion of a therapeutical gas or fluid may be used. The application of such a medium e.g. in between the two distanced inflated balloon devices may improve the surgical treatment of the patient.

According to another exemplary embodiment of the invention, the step of increasing the distance between the first balloon device and the second balloon device is carries out by translating at least one of the first balloon device and the second balloon device along a longitudinal axis of the tube of the catheter in an inflated state.

According to another exemplary embodiment of the invention, the method comprises the step of uncovering a first opening of the tube for releasing the generated micro foam or optical energy between the distanced first and second balloon device upon increasing the distance of the first and second balloon device.

In order to increase the distance of the first and second balloon device, a push mechanism or also a pull mechanism may be used in order to change the absolute position of at least one of the first and second balloon device. Therein mechanical mechanisms, electrical mechanisms, electronical mechanisms and any combination thereof may be provided. Such a mechanism may also be used to translate both of the balloon devices. If desired, also a rotational component of the movement of the movable balloon device may be caused by the presented method.

It can be seen as a gist of the invention to generate or establish a spatially separated vessel section by means of two inflatable balloon devices that are caused to perform a relative movement to each other in order to uncover an opening. Further, the present inventions allows for releasing a micro foam via said opening, which micro foam has been generated shortly before and inside the catheter. In particular for micro foam sclerosis this has several advantages. The relative movement is caused by means of the catheter. The opening is comprised by the tube of the catheter and allows for emitting for example optical energy into the spatially separated vessel section. In other words, the vessel section is spatially confined by the walls of the vessel and by the distanced first balloon device and second balloon device which may be inflated or non-inflated.

These and other aspects of the invention will become apparent from and elucidated with reference to the embodiments described hereinafter.

### BRIEF DESCRIPTION OF THE DRAWINGS

Exemplary embodiments of the invention will be described in the following drawings.
Fig. 1 schematically shows a catheter according to an exemplary embodiment of the invention.
Fig. 2 schematically shows a catheter according to an exemplary embodiment of the invention.
Fig. 3 schematically shows a catheter according to an exemplary embodiment of the invention.
Figs. 4 shows a tube for a catheter according to an exemplary embodiment of the present invention
Fig. 5 schematically shows a respective flow diagram of a method according to an exemplary embodiment of the invention.

### DETAILED DESCRIPTION OF EMBODIMENTS

Fig. 1 shows a micro foam sclerosing catheter 100 for creating a spatially separated vessel section 108 and for generating micro foam inside the catheter. The catheter comprises a tube 101 which is adapted to be inserted into the vessel. The catheter comprises a first balloon device 102 and second balloon device 103. The first balloon device and the second balloon device are movable arranged at the tube 101. The movable arrangement 104 of the at least one of the first balloon device and the second balloon device enables for a change of distance between the first balloon device and the second balloon device. Such a change of distance can be gathered from the following description, especially from the description regarding Fig. 3. The first balloon device and the second balloon device are movable along a longitudinal axis of the tube, which is symbolically shown by line 107 in Fig. 1. Furthermore, the catheter comprises a micro foam device 109. The micro foam device 109 is comprised by a catheter tube 110 which is part of the catheter. However, the micro foam device may also attached to the tube 101, such that tube 110 could be left aside. Fig. 1 shows an exemplary combination of two tubes 101 and 110. However, also other combinations are possible, like only one tube with a micro foam device 109 or a tubular system of three tubes with a micro foam device 109.

By means of the tube, the micro foam device 109 can be positioned close to the opening 200 (see Fig. 2) provided by tube 101. The micro foam device 109 may be embodied as axial section of the catheter tube 110 which comprises protrusions for generating turbulences in the therapeutic agent-gas mixture, i.e. the liquid gas mixture, for creating the micro foam inside catheter 100. The micro foam device 109 may also be embodied as a electromechanical device which sucks in the therapeutic agent-gas mixture and releases the micro foam into the catheter tube 110. Shortly thereafter the foam may be released to the subject in the separated vessel section. Therefore, a reduced amount of decay or decomposition of the micro foam can be ensured by the presented catheter. The micro foam device 109 may also be embodied as a filter or a sieve, which respectively causes turbulences in the therapeutic agent-gas mixture flowing through catheter tube 110. In addition, a gas supply line 105 is schematically shown in Fig. 1. This may also be embodied as a liquid supply line. If desired a corresponding tank may be present. The gas supply line 105 is directed towards the first balloon device 102 and to the second balloon device 103. As the tube 101 as well as the gas supply line 105 is partially covered by the first balloon device and second balloon device, the respective lines are depicted in dashed lines in the region of the balloon devices. The gas supply line supplies gas, for example inert gas, to the balloon devices in order to inflate both balloon devices. Such an inflating process may be seen in the following Fig. 2. The catheter of Fig. 1 may comprise, if desired, a flushing channel for flushing and/or sucking off media from the separated vessel section 108. Due to clarity reasons the flushing channel is depicted and explained with respect to Fig. 3.

Fig. 2 schematically shows that the balloon devices 102 and 103 are inflated to such an amount that the lateral extent of the balloon devices leads to a direct contact with the walls of the vessel 108. In other words, a sealing of the vessel is provided by means of inflating the first and second balloon devices. Said two balloon devices may be filled with gas of fluid through the catheter. As can be gathered from Fig. 2, an opening 200 is provided by tube 101. Arrow 201 depicts the movement that is caused to the first and second balloon devices 102 and 103 by means of a moving mechanism which is not shown in this figure. Such a moving mechanism or translating mechanism may be embodied in various ways. However, such a mechanism allows for change of distance between the first balloon device and the second balloon device after having sealed the vessel 108.

As can be seen in Fig. 3, the catheter 100 of Fig. 1 and Fig. 2 is depicted in a different state. Tube 101 is still inserted into the vessel 108 and an optical fiber 302 is guided through tube 101 and passes the first balloon device 102 by means of being guided through the tube. Opening 200 allows for emitting optical energy 300 into the spatially separated vessel section which is depicted with reference sign 303. Arrow 301 symbolically depicts that a rotation of the optical fiber 302 can be performed if desired. Therefore, a radial emission of optical energy may be allowed. The first balloon device 102 and the second balloon device 103 are shown in a distanced state in which they confine the spatially separated vessel section 303. Therefore, a blood flow from the upper vessel section and the lower vessel section is prevented. Thus, a protected treatment of the vessel in the vessel section 303 can be performed by, for example, applying optical energy. Further, Fig. 3 shows a flushing channel 304 for flushing and/or sucking off media from the separated vessel section. This channel may advantageously reduce side effects of a sclerosis application as only in the separated vessel section the therapeutic agent is applied. After the treatment, the remaining media like residual micro foam may be sucked away such that it does not diffuse further into the patient's body. Thus, higher concentrations of the therapeutic agent may be used for sclerosis without additional side effects. This may also reduce the treatment time and save time for the patient and the clinician. Flushing channel 304 may be arranged on the outer surface of tube 101 as shown in Fig. 3. However, it may also be provided inside of tube 101 as a separate or as an integral part of tube 101. It may also be part of catheter tube 110. Also other embodiments of the flushing channel are possible. Thus, the presented catheter combines advantages of the double balloon aspect with advantages of the micro foam generation within the catheter.

According to another exemplary embodiment the catheter of Fig, 1, 2 and 3 may be operatively connected to a flushing device and or to a sucking device such that a flushing and or sucking of material from the separated vessel section through the flushing channel are facilitated. This may be seen as a system. Further, a pump device may be operatively connected to the catheter of Fig. 1, 2 and 3 such that a pressure is applicable by the pump device to the flow channel for transporting a liquid gas mixture through the catheter and for generating the micro foam.

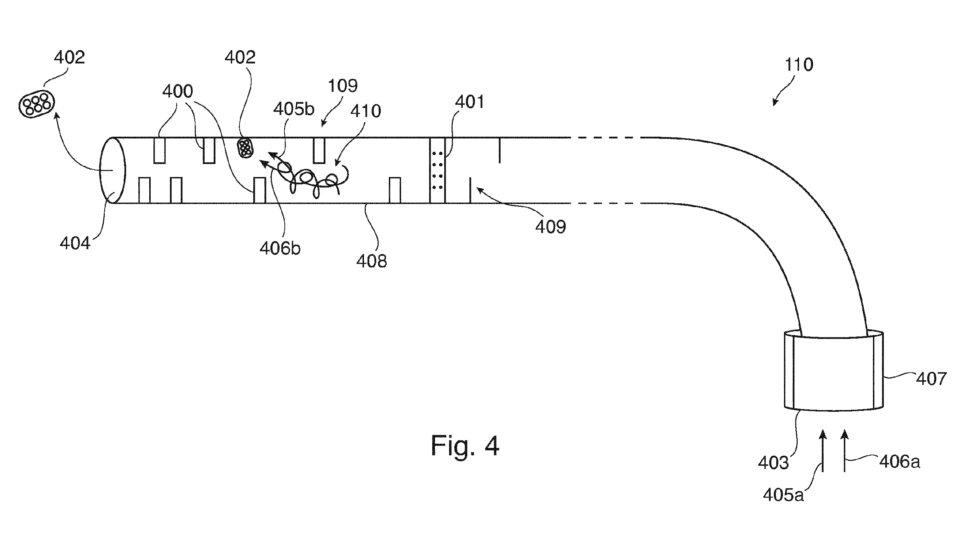

Fig. 4 shows an exemplary embodiment of a micro foam device 109 which is embodied as a plurality of protrusions 400 arranged in the inner side of tubular wall 408. This is arranged inside catheter tube 110 which can be used within the different embodiments of the invention described herein. Features of the tube 110 may also embodied at the tube 101 and vice versa. Additionally, sieve 401 is arranged in the flow channel 409 in which a liquid gas medium is guided. Thus, the flow channel 409 comprises a plurality of protrusions for creating turbulences in a liquid gas mixture flowing through the flow channel. The therapeutic agent for sclerosis 405a and the gas 406a are guided into catheter tube 110 at the end 403. Due to the sieve and the protrusions 400 protruding into the flow channel 409 turbulences 410 are caused. This leads to the generation of the desired foam 402. The therapeutic agent for sclerosis and the gas are depicted in a state of turbulences with reference signs 405b and 406b. As can be gathered from Fig. 4, the distance of adjacently positioned protrusions 400 in the flow channel decreases in the direction from the first opening 403 to the second opening 404. In another embodiment of catheter tube 110, only the protrusions 400 are present in the tube without sieve 401. In a further embodiment of catheter tube 110, only the sieve 401 is present in the tube without the protrusions 400. In any case tube catheter. Furthermore, catheter tube 110 comprises Luer Lock element 407.

The catheter tube 110 advantageously avoids that especially foam that has been created by using a liquid therapeutic drug of low concentration is partially or even totally collapsing on its way to the application. Therefore, the function of catheter tube 110 allows combining a longer catheter with micro foam sclerosis application. The presented catheter tube facilitates the administration of low concentrated foam efficiently far from where the catheter enters the body. By generating the foam on the way of the gas and liquid mixture along the catheter tube the invention enables the therapist to use less toxic low concentrated foam even on spots in a vessel that are far away from the spot of puncture. Avoiding the need to create and administer higher concentrated foam the invention enables the therapist to use smaller volumes of foam since it reaches the spot of treatment in the optimum quality of the bubbles and therefore reduces the risk of undesired side effects such as nausea, transient blindness, headache, migraine, stroke, etc. Catheter tube 110 may part of a catheter being embodied as a tubular system comprising three tubes, wherein the first tube is inside of the second and the third tube, whereas the second tube is inside the third tube, and wherein the a micro foam device for generating the micro foam in the catheter is positioned in the first tube. In this case catheter tube 110 could be the first tube. If desired, the features of a first and second balloon devices and the movable arrangement of them at the tube can be supplemented to the tube 110 of Fig. 4.

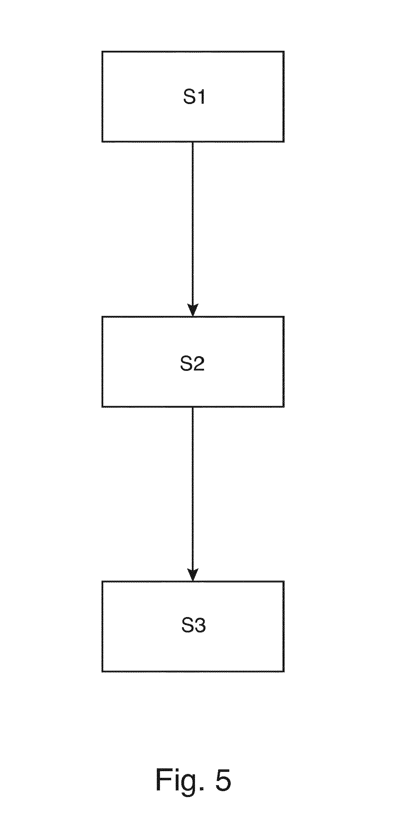

Fig 5 shows a flow diagram of a method for generating a micro foam creating a spatially separated vessel section inside of a catheter, the method comprises the steps of providing for a micro foam sclerosing catheter comprising a micro foam device for generating a micro foam inside the catheter which step his depicted with S1. The step guiding a liquid gas mixture trough the micro foam device of the catheter is shown with S2 and S3 depicts the step of generating thereby the micro foam.

## Claims

1. Micro foam sclerosing catheter (100) for creating a spatially separated vessel section (303) and for generating a micro foam inside the catheter, the catheter comprising:
a tube for inserting into a vessel,
a first balloon device (102),
a second balloon device (103),
wherein at least one of the first balloon device and the second balloon device is movably arranged at the tube,
wherein the movable arrangement (104) of the at least one of the first balloon device and the second balloon device enables for a change of distance between the first balloon device and the second balloon device, and
a micro foam device for generating a micro foam inside the catheter.

2. Micro foam sclerosing catheter according to claim 1,
wherein the first and second balloon devices are movably arranged at the tube in such a way that upon an increase of said distance between the first and second balloon devices the first and second balloon devices are positioned at opposing positions of the opening.

3. Micro foam sclerosing catheter according to one of the preceding claims, the catheter further comprising
at least one medium supply line,
wherein the at least one medium supply line is directed to the first balloon device and the second balloon device, and
wherein the first balloon device and the second balloon device are inflatable by supplying a medium to the first and the second balloon device via the medium supply line.

4. Micro foam sclerosing catheter according to one of the preceding claims, the catheter further comprising
a flushing channel for flushing and/or sucking off media from the separated vessel section.

5. Micro foam sclerosing catheter according to one of the preceding claims,
wherein the catheter is adapted to allow for sequentially inflating and distancing of the first and second balloon devices in such a way that firstly the first and the second balloon devices are inflatable in an adjacent position and in such a way that secondly the distance between the first and second balloon device is adjustable in an inflated state.

6. Micro foam sclerosing catheter according to one of the preceding claims,
wherein the catheter is embodied as a tubular system comprising three tubes, wherein the first tube is inside of the second and the third tube, whereas the second tube is inside the third tube, and
wherein the a micro foam device for generating the micro foam in the catheter is positioned in the first tube.

7. Micro foam sclerosing catheter according to one of the preceding claims,
wherein the micro foam device comprises a first opening into which a liquid and/or gas is guidable and a second opening configured to release the generated micro foam towards the created spatially separated vessel section;
wherein the micro foam device comprises a flow channel between the first and second opening; and
wherein the flow channel is configured to generate the micro foam between the first and second opening.

8. Micro foam sclerosing catheter according to claim 7,
wherein a pump device is operatively connected to the catheter such that a pressure is applicable by the pump device to the flow channel for transporting a liquid gas mixture through the catheter and for generating the micro foam.

9. Micro foam sclerosing catheter according to claim 7 or 8,
wherein the flow channel comprises a plurality of protrusions for creating turbulences in a liquid gas mixture flowing through the flow channel.

10. Micro foam sclerosing catheter according to claim 9,
wherein the protrusions are configured to cause turbulences to the liquid gas mixture such that the micro foam is generated out of the flowing liquid gas mixture.

11. Micro foam sclerosing catheter according to claims 9 or 10,
wherein a distance of adjacently positioned protrusions in the flow channel decreases in a direction from the first to the second opening.

12. Micro foam sclerosing catheter according to one of claims 9 to 11,
wherein the protrusions are embodied as protrusions which radially protrude from an inner tube surface of the tube.

13. Micro foam sclerosing catheter according to one of the preceding claims,
wherein the catheter comprises a first end configured to be grasped by a clinician,
wherein the catheter comprises a second end for releasing the micro foam into the created spatially separated vessel section, and
wherein the micro foam device is positioned at the catheter at a distance from the second end which distance is between 0 meters to 0,4 meters.

14. Catheter tube for generating a micro foam inside a catheter, the catheter tube comprising:
a micro foam device for generating a micro foam inside the catheter.

15. Method for generating a micro foam inside of a catheter, the method comprising the steps of
providing for a micro foam sclerosing catheter comprising a micro foam device for generating a micro foam inside the catheter (S1),
guiding a liquid gas mixture trough the micro foam device of the catheter (S2) and thereby generating the micro foam (S3).
